# EUROPEAN PATENT APPLICATION

(11) **EP 2 123 620 A1**
(43) Date of publication of application: **25.11.2009**
(21) Application number: 08075509.3
(22) Date of filing: 20.05.2008
(51) Int. Cl.: C07B 59/00, C07C 229/24, C07C 237/06, A61K 31/04, A61P 35/00, A61K 51/04

(54) **{F-19}-labeled L-Glutamic acid and L-Glutamine derivative (III), use thereof and method for obtaining them**

(71) Applicant: Bayer Schering Pharma Aktiengesellschaft, 13353 Berlin (DE)
(72) Inventor: Friebe, Matthias, Dr., 13509 Berlin (DE); Schmitt-Willich, Heribert, Dr., 10551 Berlin (DE); Berndt, Mathias, Dr., 12163 Berlin (DE); Koglin, Norman, Dr., 12587 Berlin (DE); Müller, Andre, Dr., 13591 Berlin (DE); Dinkelborg, Ludger, Dr., 13465 Berlin (DE); Graham, Keith, Dr., 10115 Berlin (DE)

(57) **Abstract**

The present invention relates to fluorinated glutamic acid (glutamate) and glutamine derivatives wherein the fluorine atom is 19F. The glutamic acid (glutamate) and glutamine derivatives are compound(s) of general Formula I, these are diastereoisomers and/or enantiomere derivatives.

## Description

### FIELD OF INVENTION

The present invention relates to fluorinated glutamic acid (glutamate) and glutamine derivatives wherein the fluorine atom is 19F. The glutamic acid (glutamate) and glutamine derivatives are compound(s) of general Formula I, these are diastereoisomers and/or enantiomere derivatives.

The compounds of the present invention are useful for therapy of diseases related to glutamine catabolism and the present invention further relates also to improved imaging agents useful for Fluorine-19 Magnetic Resonance Imaging (¹⁹F MRI) and as reference compounds for the identification of [F-18] glutamic acid derivatives.

### BACKGROUND ART

Glutamate is a key molecule in cellular metabolism. In humans, dietary proteins are broken down by digestion into amino acids, which serves as metabolic fuel for other functional roles in the body. A key process in amino acid degradation is transamination, in which the amino group of an amino acid is transferred to an α-ketoacid, typically catalysed by a transaminase. Glutamine has a variety of biochemical functions including:
A substrate for DNA synthesis,
Major role in protein synthesis,
Primary source of fuel for enterocytes (cells lining the inside of the small intestine),
Precursor for rapidly dividing immune cells, thus aiding in immune function,
Regulation of acid-base balance in the kidney by producing ammonium. See : http://en.wikipedia.org/wiki/Glutamine - cite note-1#cite note-1
Alternative source of fuel for the brain and helps to block cortisol-induced protein catabolism a form of fixed nitogen by heterocysts, exchanged for photosynthate from undifferentiated cyanobacterial cells.

Medina et al. (Molecular and Cellular Biochemistry 113:1-15,1992) refer to the glutamine analog, L-glutamic acid gamma-mono-hydroxamate that has demonstrated high toxicity against tumor cells in culture and "in vivo" against leukemia and B16 melanoma. Medina et al. suggest that glutaminase can be used for therapeutic use or that selective inhibition of glutamine transport by tumor cells maybe used for reduce tumor proliferation.

There is a clear need for alternative glutamine analogues that are involved in the treatment of proliferative diseases such as tumor and cancer.

It has been surprisingly found that the compounds of the invention are useful for MRI imaging as well as for therapeutic applications.

### SUMMARY

The present invention relates to fluorinated glutamic acid (glutamate) and glutamine derivatives wherein a 19F is incorporated. The compounds of the present invention are useful for MRI imaging and for treating proliferative diseases. Composition comprising compounds of the present invention are also disclosed.

The invention relates also to kit comprising new fluorinated glutamic acid (glutamate) and glutamine derivatives.

### DETAILLED INVENTION

In a first aspect, the present invention is directed to Compound(s) of general Formula I 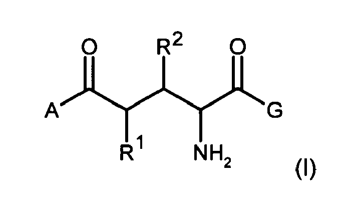 wherein
A is
a) Hydroxyl,
b) branched or unbranched C₁-C₅ alkoxy,
c) branched or unbranched Hydroxy C₁-C₅ Alkoxy,
d) branched or unbranched O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L,
h) O-L or
i) O-Z,
G is
a) Hydroxyl,
b) O-Z
b) branched or unbranched O-C₁-C₅ Alkyl,
c) branched or unbranched O-C₂-C₅ Alkenyl,
d) branched or unbranched O-C₁-C₅ Alkyl-(O-C₁-C₄ alkyl)ₙ-C₁-C₄ alkyl or
e) branched or unbranched O-C₂-C₅ Alkinyl,
and R¹ and/or R², independently separate are
a) Hydrogen,
b) branched or unbranched ¹⁹F-C₁-C₁₀ Alkoxy,
c) branched or unbranched ¹⁹F-C₁-C₁₀ Alkyl,
d) branched or unbranched ¹⁹F-C₂-C₁₀ Alkenyl,
e) branched or unbranched ¹⁹F-C₂-C₁₀ Alkinyl,
f) substituted or unsubstituted ¹⁹F-C₆-C₁₀ mono- or bicyclic Aryl,
g) substituted or unsubstituted ¹⁹F-C₅-C₁₀ mono- or bicyclic Heteroaryl,
h) substituted or unsubstituted ¹⁹F-C₃-C₆ Cyclo-Alkyl,
i) Hydroxyl,
j) branched or unbranched C₁-C₅ Alkyl or
k) branched or unbranched C₁-C₅ Alkoxy
   with the proviso that one of the substituent R¹ or R² comprises ¹⁹F atom and the other substituent comprises no ¹⁹ F atom,
L is
a) branched or unbranched C₁-C₅ Alkyl,
b) branched or unbranched C₂-C₅ Alkenyl,
c) branched or unbranched C₁-C₅ Alkyl-(O-C₁-C₄ alkyl)ₙ-O-C₁-C₄ alkyl oder
d) branched or unbranched C₂-C₅ Alkinyl,
   Z is a metal ion equivalent ,
   n = 0, 1, 2 or 3 and
   pharmaceutical salt, diastereomere and enantiomere thereof.

In a preferred embodiment of compounds of Formula I, A is Hydroxyl, branched or unbranched C₁-C₅ alkoxy or NH₂.
In a more preferred embodiment, A is ethoxy.

In a preferred embodiment of compounds of Formula I, G is Hydroxyl or branched or unbranched C₁-C₅ alkoxy.
In a more preferred embodiment, G is methoxy.

In a preferred embodiment of compounds of Formula I, R¹ or R² is
a) branched or unbranched ¹⁹F-C₁-C₁₀ Alkoxy,
a) branched or unbranched ¹⁹F-C₁-C₁₀ Alkyl,
b) branched or unbranched ¹⁹F-C₂-C₁₀ Alkenyl,
c) branched or unbranched ¹⁹F-C₂-C₁₀ Alkinyl.

In a preferred embodiment of compounds of Formula I, R¹ or R² is
a) branched or unbranched ¹⁹F-C₁-C₅ Alkoxy, more preferably ¹⁹F-C₃ Alkoxy
b) branched or unbranched ¹⁹F-C₁-C₅ Alkyl, more preferably ¹⁹F-C₃ Alkyl,
c) branched or unbranched ¹⁹F-C₂-C₅ Alkenyl, more preferably ¹⁹F-C₃ Alkenyl,
d) branched or unbranched ¹⁹F-C₂-C₅ Alkinyl. more preferably ¹⁹F-C₃ Alkinyl.

In a more preferred embodiment of compounds of Formula I, R¹ or R² Is
a) branched or unbranched ¹⁹F-C₆-C₁₀ Alkoxy, more preferably ¹⁹F-C₆ Alkoxy,
b) branched or unbranched ¹⁹F-C₆-C₁₀ Alkyl, more preferably ¹⁹F-C₆ Alkyl,
c) branched or unbranched ¹⁹F-C₆-C₁₀ Alkenyl, more preferably ¹⁹F-C₆ Alkenyl,
d) branched or unbranched ¹⁹F-C₆-C₁₀ Alkinyl, more preferably ¹⁹F-C₆ Alkinyl.

In a further preferred embodiment of compounds of Formula I, R¹ or R² Is
a) substituted or unsubstituted ¹⁹F-C₆-C₁₂ mono- or bicyclic Aryl,
b) substituted or unsubstituted ¹⁹F-C₅-C₁₂ mono- or bicyclic Heteroaryl,
c) substituted or unsubstituted ¹⁹F-C₃-C₆ Cyclo-Alkyl.

In a further preferred embodiment of compounds R¹ is ¹⁹F and R² is hydrogen.

In a preferred embodiment, Z is selected from Mg²⁺, Ca²⁺, Na⁺ und K⁺ .
In a more preferred embodiment Z is Na⁺ .

In a preferred embodiment, n = 0, 1 or 2.
In a more preferred embodiment, n = 0, 1.

The compounds of the present invention are D- or L- diastereoisomere (position C₂) and R- or S-enantiomere (position C₄).
In a preferred embodiment the compounds of Formula I are L- diastereoisomere and/or S-enantiomere.

Of this group, a preferred subgroup of compounds is selected from the following: 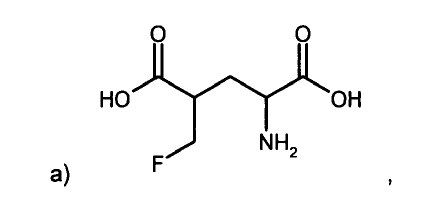 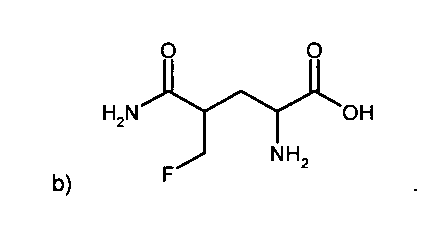 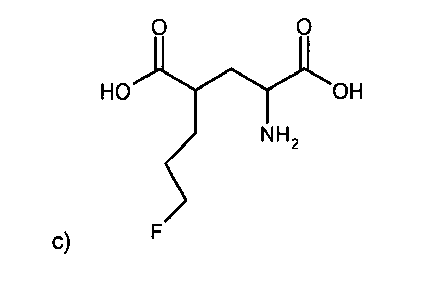 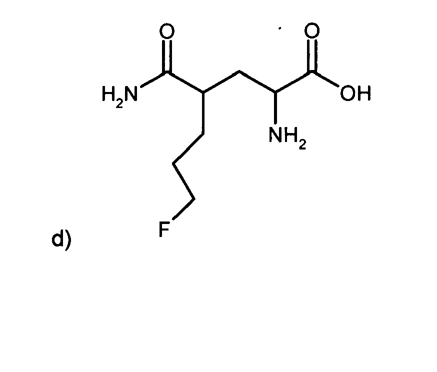 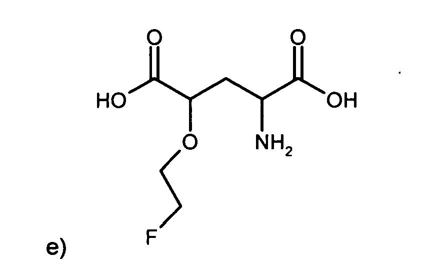 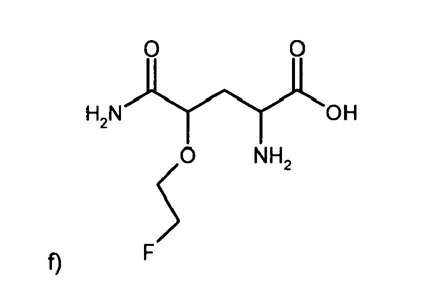 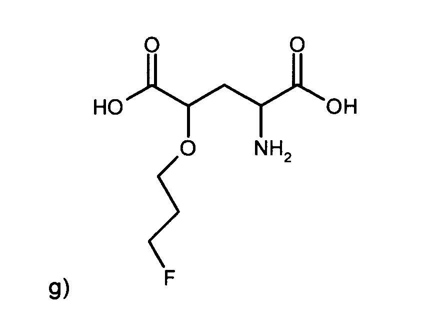 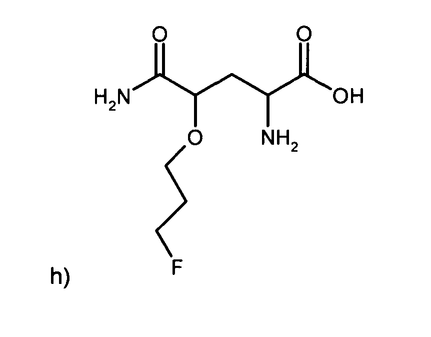 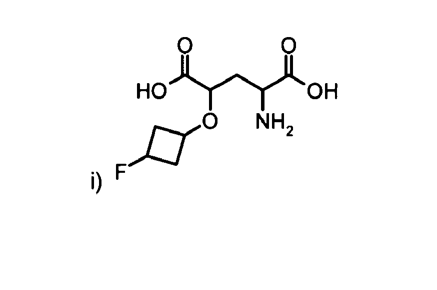 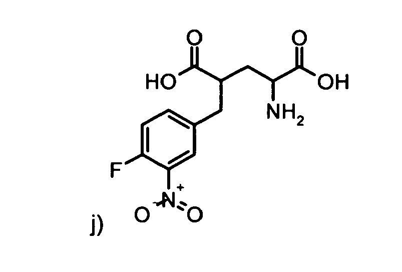 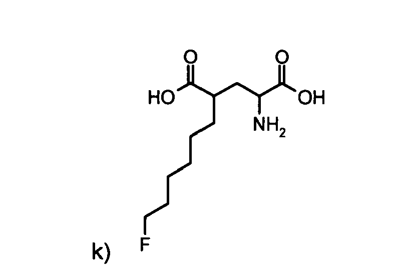 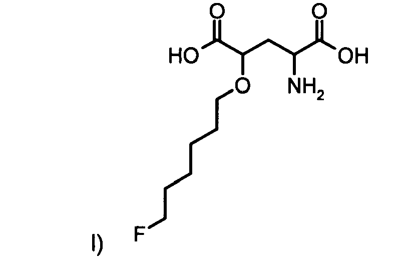 and
l) 2-Amino-4-[F-19]fluoroglutamin.

In a second aspect, the present invention is directed to one compound or more compounds of general Formula I and a pharmaceutical acceptable carrier.

In a third aspect, the present invention is directed to a method for obtaining compound of formula I by reacting a non-fluorinated compound of formula I with a fluorine atom ¹⁹F or moiety comprising fluorine atom ¹⁹F .

In a fourth aspect, the present invention is directed to a compound of formula I for use as a medicament.

In a preferred embodiment, the present invention relates to the use of compound of formula I for the manufacturing of a medicament for use as an inhibitor of proliferative diseases. Preferably, proliferative diseases are characterized by metastasis or tumor.

More preferably proliferative disease is a disease developing malignant tumor selected from malignant lymphoma, pharyngeal cancer, lung cancer, liver cancer, bladder tumor, rectal cancer, prostatic cancer, uterine cancer, ovarian cancer, breast cancer, brain tumor, and malignant melanoma.

Further the compound of formula I is to be administered orally, parenterally, rectally, or locally.

In a more preferred embodiment the medicament is for treating, preventing or alleviating proliferative diseases growth.

In a preferred embodiment, the present invention relates a method for treating proliferative diseases comprising administering to an individual in need thereof a therapeutically effective amount of compound of formula I as defined above.

In a fifth aspect, the present invention is directed to compound of formula I for use as imaging agent.

In a preferred embodiment, the present invention relates to the use of compound of formula I for the manufacturing of a imaging agent for imaging proliferative diseases.

Preferably, proliferative diseases are characterized by metastasis or tumor.

More preferably proliferative disease is a disease developing malignant tumor selected from malignant lymphoma, pharyngeal cancer, lung cancer, liver cancer, bladder tumor, rectal cancer, prostatic cancer, uterine cancer, ovarian cancer, breast cancer, brain tumor, and malignant melanoma.

Further the compound of formula I is to be administered orally, parenterally, rectally, or locally.

In a more preferred embodiment the imaging agent is a Magnetic Resonance Imaging (MRI) agent.

In a preferred embodiment, the present invention relates to a method for imaging proliferative diseases more preferably metastasis and tumor comprising administering to an individual in need thereof a therapeutically effective amount of compound of formula I.

In a sixth aspect, the present invention is directed to a kit comprising compound of formula I as defined above. The kit contains further a F-18-PET imaging agent precursor.

In a seven aspect, the present invention is directed to the use of the compounds of formula I as defined above for the identification of F-18-PET-Tracer. The compounds of formula I are useful as a competition agent during PET imaging (reference compound) and for identifying new F-18-PET-Tracer .

### EXAMPLES

Unless defined otherwise, all technical and scientific terms used herein have the same meaning as commonly understood by one of the ordinary skill in the art to which this invention belongs. Although any methods and materials similar or equivalent to those described herein can be used in the practice or testing of the present invention, the preferred methods and materials are described below. The following schematic examples relates to the preparation of a compounds according to Formula I. The examples presented below are not to be understood as to limit the invention to the methods exemplified herein.

### DEFINITIONS

The term "therapeutically effective amount" as used herein refers to that amount of a compound of the invention which, when administered to an individual in need thereof, is sufficient to effect treatment, as defined below, for metastasis. The amount which constitutes a "therapeutically effective amount" will vary depending on the compound, the disease and its severity, and the age of the human to be treated, but can be determined routinely by one of ordinary skill in the art having regard to his own knowledge and to this disclosure.

"Treating" or "treatment" as used herein refers to the treatment proliferative diseases and include:
(i) preventing the disease from recurring in an individual, in particular, when such individual is in need of further medicamentous treatment after a previous surgical or medicamentous therapy;
(ii) inhibiting the disease, i.e., arresting its development; or
(iii) relieving the disease, i.e., causing regression of the disease.

The term "alkyl" as used herein refers to straight or branched alkyl groups, e. g., methyl, ethyl, propyl, isopropyl, *n*-butyl, *t*-butyl, *n*-pentyl, neopentyl, heptyl, or decyl. Alkyl groups can be perfluorated or substituted by one to five substituents selected from the group consisting of halogen, hydroxy, C₁-C₄ alkoxy, or C₆-C₁₂ aryl (which can be substituted by one to three halogen atoms).
The term "alkenyl" as used herein refers to a straight or branched chain monovalent or divalent radical, containing at least one double bond and having from two to ten carbon atoms, *e*.*g*., ethenyl, prop-2-en-1-yl, but-1-enyl, pent-1-enyl, penta-1,4-dienyl, and the like.

The term "alkynyl" as used herein refers to a substituted or unsubstituted straight or branched chain monovalent or divalent radical, containing at least one triple bond and having from two to ten carbon atoms, *e*.*g*., ethynyl, prop-1-ynyl, but-1-ynyl, pent-1-ynyl, pent-3-ynyl, and the like.

Alkenyl and alkenyl groups can be substituted by one or more substituents selected from the group consisting of halogen, hydroxy, alkoxy, -CO₂H, -CO₂Alkyl, -NH₂, -NO₂, -N₃, -CN, C₁-C₂₀ acyl, or C₁-C₂₀ acyloxy.

The term "aryl" as used herein refers to an aromatic carbocyclic or heterocyclic moiety containing five to 14 ring atoms, e.g., phenyl, naphthyl, furyl, thienyl, pyridyl, pyrazolyl, pyrimidinyl, oxazolyl, pyridazinyl, pyrazinyl, chinolyl, or thiazolyl. Aryl groups can be substituted by one or more substituents selected from the group consisting of halogen, hydroxy, alkoxy, -CO₂H, -CO₂Alkyl, -NH₂, Alkyl-NH₂, C₁-C₂₀ alkyl-thiolanyl, -NO₂, -N₃, -CN, C₁-C₂₀ alkyl, C₁-C₂₀ acyl, or C₁-C₂₀ acyloxy. The heteroatoms can be oxidized, if this does not cause a loss of aromatic character, e. g., a pyridine moiety can be oxidized to give a pyridine N-oxide.
The rings can be substituted by one or more substituents selected from the group consisting of halogen, hydroxy, alkoxy, -CO₂H, -CO₂Alkyl, -NH₂, -NO₂, -N₃, -CN, C₁-C₂₀ alkyl, C₁-C₂₀ acyl, or C₁-C₂₀ acyloxy.

Typically, an effective amount of an imaging agent formulation comprising the F magnetic resonance imaging agent and a pharmaceutically acceptable carrier is administered to the patient, and the patient, or a portion of the patient, is imaged. The term "effective amount", as used herein, denotes a non-toxic amount sufficient to enhance or alter the MRI image obtained, more particularly, an amount which permits better visualization of the organs and/or tissues being imaged.
Preferably the patient is a mammal; most preferably the patient is a human.
The ¹⁹F magnetic resonance imaging agents of the present invention may be variously administered by any suitable route, including, for example, orally, for imaging of the upper gastrointestinal tract; rectally, for imaging of the lower gastrointestinal tract including the colon; nasally, for imaging of the nasal and communicating passages; vaginal, for imaging of the fallopian tubes and communicating passages; parenteral (including subcutaneous, intramuscular, intravenous, intradermal and pulmonary), for imaging of internal organs, tissues, tumors, and the like. It will be appreciated that the preferred route will vary with the organs or tissues to be imaged. Preferred routes of administration include parenteral and oral, more preferably intravenous.
While it is possible for the imaging agent to be administered alone, it is preferable to present it as a pharmaceutical formulation comprising at least one imaging agent compound, together with one or more pharmaceutically acceptable carriers, such as diluents or excipients which may include, for example, fillers, extenders, wetting agents, disintegrants, surface-active agents, or lubricants, depending on the nature and mode of administration and the dosage forms. Each carrier must be "acceptable" in the sense of being compatible with the other ingredients of the formulation and not injurious to the patient. The pharmaceutical formulation may optionally include other diagnostic or therapeutic agents. Techniques and formulations may be found, for example, in Remington's Pharmaceutical Sciences. Mack Publishing Co., Easton, PA. (latest edition).

Formulations of the present invention suitable for oral administration may be presented as an aqueous solution. Alternatively, formulations can be administered as capsules, cachets or tablets, each containing a predetermined amount of the imaging agent; powder; granules; or paste.
Formulations suitable for parenteral administration include aqueous isotonic sterile injection solutions which may contain anti-oxidants, buffers, bacteriostats and solutes which render the formulation isotonic with the blood of the intended recipient; and aqueous sterile suspensions which may include suspending agents and thickening agents, and liposomes or other microparticulate systems which are designed to target the compound to one or more tissues or organs. The formulations may be presented in unit-dose or multi-dose sealed containers, for example, ampules and vials, and may be stored in a freeze-dried (lyophilized) condition requiring only the addition of the sterile liquid carrier, for example water, for injections immediately prior to use. Extemporaneous injection solutions and suspensions may be prepared from sterile powders, granules or tablets.
It should be understood that in addition to the ingredients particularly mentioned above, the formulations of this invention may include other agents conventional in the art having regard to the type of formulation in question, for example, those suitable for oral administration may include such further agents as sweeteners, thickeners and flavoring agents.
The F magnetic resonance imaging agents of the present invention may also be presented for use in the form of veterinary formulations, which may be prepared, for example, by methods that are conventional in the art.
For effective F MRI, dosages of the ¹⁹F magnetic resonance imaging agent will depend on the spin density, flow (diffusion and perfusion), susceptibility, and relaxivity (TI and T2) of the imaging agent formulation. Dosages of' F containing imaging agents may be conveniently calculated in milligrams of ¹⁹F per kilogram of patient (abbreviated as mg ⁹F/kg). For example, for parenteral administration, typical dosages may be from about 50 to about 1000 mg ¹⁹F kg, more preferably from about 100 to about 500 mg ¹⁹F/kg.
For methods of continuous administrations (e.g., intravenous), suitable rates of administration are known in the art. Typical rates of administration are about 0.5 to 5 mL of formulation per second, more preferably about 1-3 mUs. Imaging may begin before or after commencing administration, continue during administration, and may continue after administration. It will be appreciated that dosages, dosage volumes, formulation concentrations, rates of administration, and imaging protocols will be individualized to the particular patient and the examination sought, and may be determined by an experienced practitioner. Guidelines for selecting such parameters are known in the art (see, inter alia, Katzberg, 1992, supra).

The usefulness and efficiency of chemical compounds as contrast agents depends on their ability to exhibit a predictable and desirable biodistribution and metabolism in vivo. Their behavior in vivo depends on parameters such as molecular weight, charge, osmolality, hydrophobicity, partition coefficient, susceptibility to metabolic breakdown, and tissue or organ targeting efficiency. In order to improve their solubility and/or biodistribution, many contrast agents are used in conjunction with delivery systems such as emulsions, liposomes, and microparticles.

### Examples:

### Beispiel 1

### (2S,4S)-2-Amino-4-(3-fluoropropyl)-pentane dioic acid

### a) (2S,4S)-4-allyl-2-tert-butoxycarbonylamino-pentane dioic acid dimethyl ester

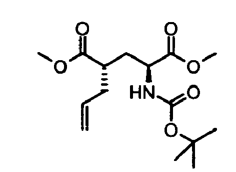

11.01 g (40 mmol) of Boc-Glutamic acid dimethylester (Advanced Chemtech) were dissolved in 160 mL tetrahydrofurane (THF) and cooled to -70°C. 88 mL (88 mmol) of a 1 M solution of Lithium-bis(trimethylsilyl)amide in THF was added dropwise over a time-period of 1 hr at -70 °C and was further stirred for 2 hr at -70 °C. Subsequently, 14.52 g (120 mmol) of allylbromide were added dropwise over 2 hr, then the cooling bath was removed and 200 mL of 2 N hydrochloric acid and 400 mL of ethylacetate were added. The organic phase was separated, washed neutral with water, dried over sodium sulphate, filtered and reduced in volume by evaporation. The crude material was chromatographed with hexane/ ethylacetate on silica gel. The obtained product fractions were combined and the solvents were evaporated to dryness.
Yield: 3.3 g (26 %)

### Elemental analysis:

| | | | |
|---|---|---|---|
| calc.: | C 57.13 | H 7.99 | N 4.44 |
| found: | C 56.97 | H 8.12 | N 4.30 |

### b) (2S,4S)-2-tert-Butoxycarbonylamino-4-(3-hydroxypropyl)-pentan dioic acid dimethyl ester

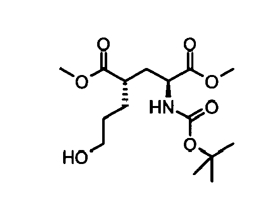

3.15 g (10 mmol) of product **1a** was dissolved in 50 mL THF and was cooled in an ice bath. 13.3 mL of 1 M Diboran/THF-complex in THF was added dropwise over a time period of. 20 min under a nitrogen flow and ice cooling. The mixture was stirred for 1 hr on ice, and over night at room temperature. Subsequently, 15 mL of 1 N sodium hydroxide followed by 13.3 mL of 30% aqueous hydrogen peroxide solution were added dropwise. The reaction mixture was diluted with water after 30 min, the THF was distilled off and the aqueous remainder was extracted with ethyl acetate. The organic phase was separated, washed neutral with water, dried over sodium sulphate, filtered and reduced in volume by evaporation on an evaporator. The crude product was purified by column chromatography using a gradient of hexane/ ethylacetate on silica gel. The product fractions were combined and the solvents were evaporated to dryness.
Yield: 0.6 g (18 %)

### Elemental analysis:

| | | | |
|---|---|---|---|
| calc.: | C 54.04 | H 8.16 | N 4.20 |
| found: | C 53.88 | H 8.25 | N 4.39 |

### c) (2S,4S)-2-tert-Butoxycarbonylamino-4-(3-fluoropropyl)-pentane dioic acid dimethyl ester

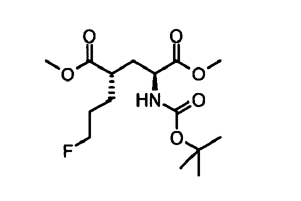

0.33 g (1 mmol) of hydroxy compound **1b** was dissolved in 15 mL of dichloro methane and cooled in an ice bath. The reaction mixture was stirred for 1 hr on the ice bath after addition of 0.32 g (2 mmol) Diethylaminosulphurtrifluoride (DAST). Then the mixture was washed with water and the organic phase was dried over sodium sulphate, filtered and reduced in volume by evaporation on an evaporator. The raw material was chromatographed in hexane/ ethyl acetate on silica gel. The product fractions were combined and the solvents were removed to dryness by evaporation.
Yield: 25 mg (8 %)

### Elemental analysis:

| | | | | |
|---|---|---|---|---|
| calc.: | C 53.72 | H 7.81 | F 5.66 | N 4.18 |
| found: | C 53.55 | H 7.94 | F 5.21 | N 4.37 |

### d) (2S,4S)-2-Amino-4-(3-fluoropropyl)-pentane dioic acid

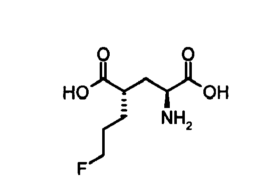

23.5 mg (0,07 mmol) of fluorinated compound **1d** was dissolved in 2 mL THF, supplemented with 1 mL of 1 N sodium hydroxide and stirred for 4 hr at room temperature. Subsequently, the mixture was reduced in volume to dryness. The remainder was re-dissolved in. 20 mL of 3 N HCl/ diethylether, stirred over night, reduced in volume by evaporation and re-distilled with diethylether repeatedly. The crude material was chromatographed with a water/ methanol-gradient on C18-silica gel. The product fractions were combined and reduced in volume to dryness.
Yield: 4 mg (27 %)

### Elemental analysis (calculated for water-free compound):

| | | | | |
|---|---|---|---|---|
| calc.: | C 46.37 | H 6.81 | F 9.17 | N 6.76 |
| found: | C 46.11 | H 7.02 | F 8.87 | N 6.93 |

## Claims

**1.** Compound of the general Formula I 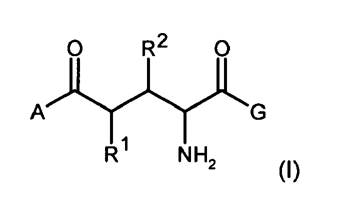 wherein
A is
a) Hydroxyl,
b) branched or unbranched C₁-C₅ alkoxy,
c) branched or unbranched Hydroxy C₁-C₅ Alkoxy,
d) branched or unbranched O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L,
h) O-L or
i) O-Z,
and G is
a) Hydroxyl,
b) O-Z
b) branched or unbranched O-C₁-C₅ Alkyl,
c) branched or unbranched O-C₂-C₅ Alkenyl,
d) branched or unbranched O-C₁-C₅ Alkyl-(O-C₁-C₄ alkyl)ₙ-O-C₁-C₄ alkyl or
e) branched or unbranched O-C₂-C₅ Alkinyl,
and R¹ and/or R² , independently separate are
c) Hydrogen,
d) ¹⁹F,
e) branched or unbranched ¹⁹F-C₁-C₁₀ Alkoxy,
f) branched or unbranched ¹⁹F-C₁-C₁₀ Alkyl,
g) branched or unbranched ¹⁹F-C₂-C₁₀ Alkenyl,
h) branched or unbranched ¹⁹F-C₂-C₁₀ Alkinyl,
i) substituted or unsubstituted ¹⁹F-C₆-C₁₀ mono- or bicyclic Aryl,
j) substituted or unsubstituted ¹⁹F-C₅-C₁₀ mono- or bicyclic Heteroaryl,
k) substituted or unsubstituted ¹⁹F-C₃-C₇ Cyclo-Alkyl,
l) Hydroxyl,
m) branched or unbranched C₁-C₅ Alkyl or
n) branched or unbranched C₁-C₅ Alkoxy
with the proviso that one of the substituent R¹ or R² comprises ¹⁹F atom and the other substituent comprises no ¹⁹ F atom,
and L is
a) branched or unbranched C₁-C₅ Alkyl,
b) branched or unbranched C₂-C₅ Alkenyl,
c) branched or unbranched C₁-C₅ Alkyl-(O-C₁-C₄ alkyl)ₙ-O-C₁-C₄ alkyl oder
d) branched or unbranched C₂-C₅ Alkinyl,
and Z is a metal ion equivalent ,
with the meaning of n = 0, 1, 2 oder 3,
and
pharmaceutical salt, diastereomere and enantiomere thereof.

**2.** Compound according to claim 1 wherein R¹ is ¹⁹F and R² is hydrogen.

**3.** Compound according to claim 1 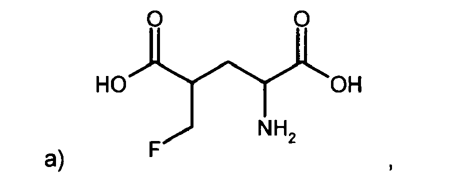 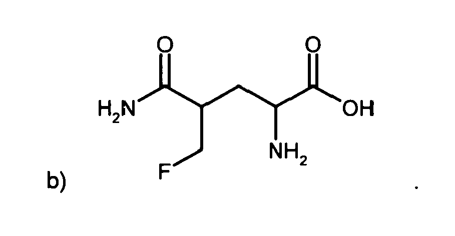 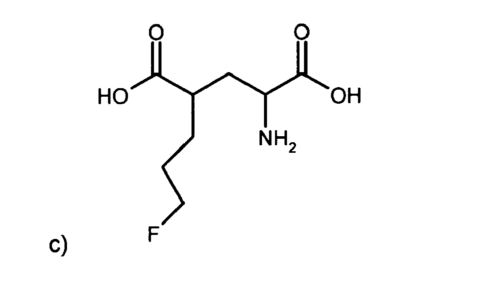 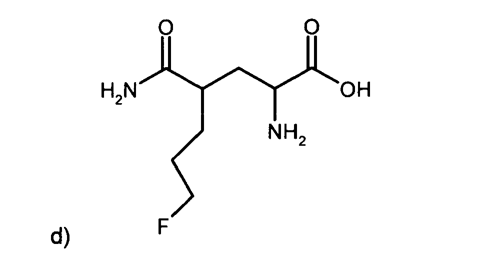 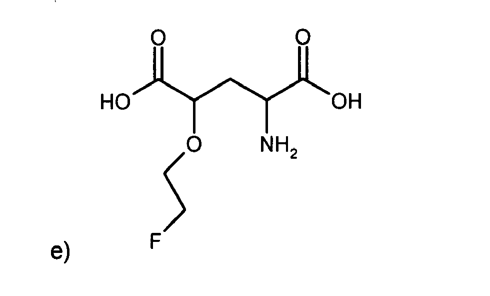 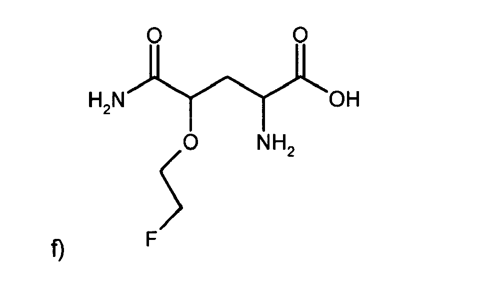 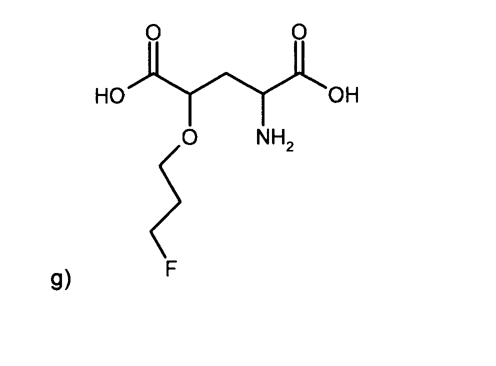 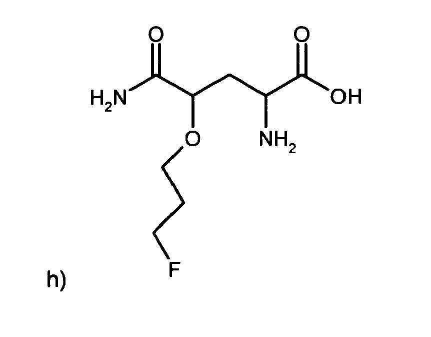 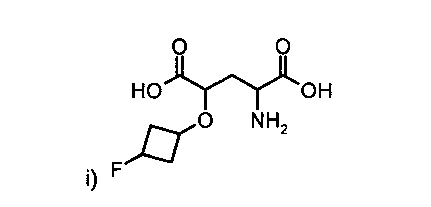 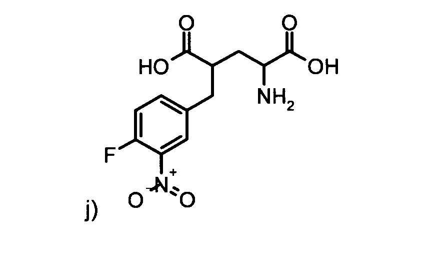 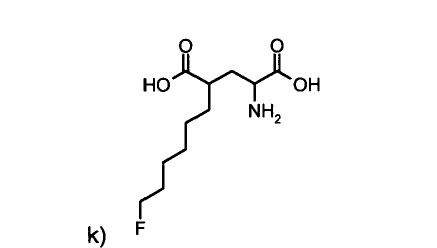 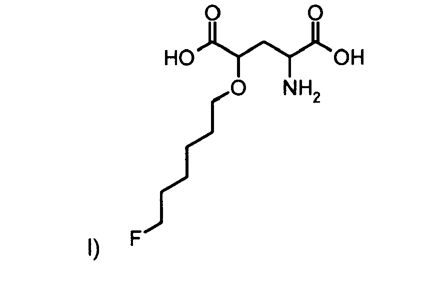 and
l) 2-Amino-4-[F-19]fluoroglutamin

**4.** Pharmaceutical composition comprising one compound or more compounds of general Formula I and a pharmaceutical acceptable carrier.

**5.** Method for obtaining compound(s) of general Formula I by reacting a non-fluorinated compound of formula I with a fluorine atom or derivative.

**6.** Compound(s) of of general Formula I according to claims 1 to 3 for use as a medicament.

**7.** Use of Compound(s) of of general Formula I according to claims 1 to 3 for the preparation of a medicament for use as an inhibitor of proliferative diseases in a patient.

**8.** Use according to claim 7 wherein the medicament is for treating, preventing or alleviating proliferative diseases.

**10.** A method for treating proliferative diseases comprising administering to an individual in need thereof a therapeutically effective amount of Compound(s) of of general Formula I as defined in any one of claims 1 to 3.

**11.** The use or the method according to any one of claims 7 to 10, wherein the compound of formula I is to be administered orally, parenterally, rectally, or locally.

**12.** Compound(s) of general Formula I according to claims 1 to 3 for use as imaging agent.

**13.** Use of Compound(s) of general Formula I according to claims 1 to 3 for the manufacturing of an imaging agent for imaging proliferative diseases in a patient.

**14.** Use according to claim 13 wherein the imaging agent is a Magnetic Resonance Imaging (MRI) agent.

**15.** A method for imaging proliferative diseases comprising administering to an individual in need thereof a therapeutically effective amount of Compound(s) of of general Formula I as defined in any one of claims 1 to 3.

**16.** The use or the method according to any one of claims 6 to 15, wherein the compound of formula I is to be administered orally, parenterally, rectally, or locally.

**17.** Kit comprising Compound(s) of general Formula I as defined in any one of claims 1 to 3.
